# EUROPEAN PATENT APPLICATION

(11) **EP 4 455 189 A1**
(43) Date of publication of application: **30.10.2024**
(21) Application number: 22911391.5
(22) Date of filing: 23.12.2022
(51) Int. Cl.: C08G 77/20, A61K 8/81, A61K 8/89, A61K 8/895, A61Q 1/12, C08F 290/14, C08F 299/08, C08G 77/38, C08J 3/12, C08L 101/00, C08L 101/16

(54) **VINYL-MODIFIED ORGANOPOLYSILOXANE, AND RADICAL POLYMERIZABLE POLYMER OR RADICAL POLYMERIZABLE COPOLYMER USING SAME AS RAW MATERIAL**

(30) Priority: 24.12.2021 JP 2021210862
(71) Applicant: Dow Toray Co., Ltd., Tokyo 140-8617 (JP)
(72) Inventor: Tan Liyi, Ichihara-shi Chiba 299-0108 (JP); SUGIURA Tsunehito, Ichihara-shi Chiba 299-0108 (JP); WAKITA Mari, Ichihara-shi Chiba 299-0108 (JP); TANIGUCHI Hiroko, Ichihara-shi Chiba 299-0108 (JP); KANZAKI Yasue, Ichihara-shi Chiba 299-0108 (JP)
(74) Representative: Murgitroyd & Company
(86) International application number: PCT/JP2022/047559
(87) International publication number: WO 2023/120689

(57) **Abstract**

PROBLEM: The present invention provides a vinyl-modified organopolysiloxane useful as a raw material for radical polymerization reactions, especially for biodegradable polymers or copolymers, as well as a method for producing the same. Furthermore, the present invention provides a radical polymerizable polymer or radical polymerizable copolymer in which the vinyl-modified organopolysiloxane is at least a portion of the starting raw material, as well as uses thereof and a method of production.

SOLUTION: A vinyl-modified organopolysiloxane having two or more vinyl-modified groups (R^{Ac}) expressed by -R¹-C(=O)-O-CH=CHR² (where R¹ is a divalent organic group having 4 to 20 carbon atoms, R² is a hydrogen atom or a methyl group) bonded to a silicon atom in the molecule and having a polyorganosiloxane structure; a radical polymerizable polymer or radical polymerizable copolymer (especially silicone elastomer particles) made therefrom; and use thereof.

## Description

### TECHNICAL FIELD

The present invention relates to vinyl-modified organopolysiloxanes having a plurality of vinyl-modified groups (functional groups having a vinyloxycarbonyl structure via a spacer of a certain chain length) attached to a specific silicon atom in the molecule, that is useful as a raw material for radical polymerization reactions, and to a method for production thereof by transvinylation of carboxylate-modified groups by vinyl acetate. The present invention also relates to radical polymerizable polymers or radical polymerizable copolymers (including, in particular, silicone elastomer particles) derived from these vinyl-modified organopolysiloxanes, as well as applications, manufacturing methods and uses thereof.

### BACKGROUND ART

Organosilicon compounds containing one vinyloxycarbonyl group in a molecule are well known, and since the vinyloxycarbonyl group is radical polymerizable, they are known to be used as polymer raw materials by homopolymerization or copolymerization reactions (for example, see Patent Document 1 and Non-Patent Document 1). However, while so-called monomer raw materials are disclosed in these documents, compounds having a plurality of vinyloxycarbonyl groups in the molecule via spacers with a specific chain length and containing an organopolysiloxane structure are not specifically disclosed.

On the other hand, in recent years, polymer or copolymer materials that continue to remain in nature without being decomposed at least for a short period of time, including the so-called microplastic problem, have begun to be avoided industrially. There is concern that polymers, copolymers, and gels or particles thereof, which are radical polymerized monomer materials having radical polymerizable functional groups such as vinyloxycarbonyl groups attached to a silicon atom via short spacers with three or fewer carbon atoms will not decompose, at least in a short period of time. Therefore, in order to reduce the risk to the global environment, there is a strong need for reactive raw materials having polymerization reaction products that are biodegradable and can be easily produced industrially.

On the other hand, focusing on the issue of biodegradability inherent in conventional silicone elastomer particles, the present applicant has proposed, as described in Patent Document 2, a silicone elastomer particle having as a main component an organopolysiloxane having at least three organic groups containing (meth)acryloxy groups and having a structure crosslinked by a divalent organic group having a substructure formed by radical polymerization of vinyl acetate. The silicone elastomer particles can be expected to have a high degree of biodegradability, have suppressed aggregation properties over time as compared with conventional silicone elastomer particles, and provide a smaller average secondary particle diameter. Therefore, these silicone elastomer particles have excellent dispersibility, and have excellent handling workability as a cosmetic raw material, storage stability, and blending stability in a system.

However, organopolysiloxanes containing organic groups containing (meth)acryloxy groups still leave room for improvement in terms of industrial productivity and structural optimization as radical polymerizable raw materials in biodegradable polymers or copolymers.

### RELATED ART DOCUMENTS

### Patent Documents

Patent Document 1: Japanese Unexamined Patent Application Publication H01-316388 A
Patent Document 2: International Patent Application PCT/JP 2021/46142

### Non-Patent Documents

Non-patent Document 1: Ego Proizvodnykh (1968), 291-4. Language: Russian, Database: CAPLUS

### SUMMARY OF THE INVENTION

### PROBLEM TO BE SOLVED BY THE INVENTION

An object of the present invention is to solve the aforementioned problem, and provide a vinyl-modified organopolysiloxane useful as a raw material for radical polymerization reactions, especially for biodegradable polymers or copolymers, as well as a method for producing the same. Furthermore, the present invention provides a radical polymerizable polymer or radical polymerizable copolymer in which the vinyl-modified organopolysiloxane is at least a portion of the starting raw material, as well as uses thereof and a method of production.

### MEANS FOR SOLVING THE PROBLEM

In order to solve the above problem and as a result of extensive study, the present inventors have discovered that the aforementioned problems can be solved by a vinyl-modified organopolysiloxane having 2 or more vinyl-modified groups (R^{Ac}) expressed by

-R¹-C(=O)-O-CH=CHR²

(wherein R¹ is a divalent organic group having 4 to 20 carbon atoms and R² is a hydrogen atom or a methyl group), bonded to a silicon atom in each molecule; and
having a polyorganosiloxane structure expressed by

   -(R³₂SiO)ₙ-
(wherein R³ is an alkyl group with 1 to 20 carbon atoms, either unsubstituted or substituted with a halogen atom, an aryl group with 6 to 22 carbon atoms, or a hydroxyl group, and n is a number in the range 1 to 1000).

Similarly, the present inventors found that the above problem can be solved by a method for producing the vinyl-modified organopolysiloxane, including a step of performing a transvinylation reaction of a specific carboxylate-modified organopolysiloxane with a vinyl carboxylate compound in the presence of a palladium metal catalyst, and thereby achieved the present invention.

The present inventors also found that the aforementioned problem can be solved by a radical polymerizable polymer or radical polymerizable copolymer, especially silicone elastomer particles, containing the aforementioned vinyl-modified organopolysiloxane as at least a portion of the starting raw material, and thereby achieved the present invention. Furthermore, the present inventors found that the above issues can be solved by cosmetic raw materials, organic resin additives, cosmetic materials, and organic resins containing the radical polymerizable polymer or radical polymerizable copolymer, and thereby achieved the present invention.

### EFFECT OF THE INVENTION

The present invention provides a vinyl-modified organopolysiloxane useful as a raw material for radical polymerization reactions, especially for biodegradable polymers or copolymers, as well as a method for producing the same. Furthermore, the present invention provides a radical polymerizable polymer or radical polymerizable copolymer in which the vinyl-modified organopolysiloxane is at least a portion of the starting raw material, as well as uses thereof and a method of production.

### PREFERRED MODE FOR CARRYING OUT THE INVENTION

### Vinyl-modified organopolysiloxane

The vinyl-modified organopolysiloxane of the present invention has two or more vinyl-modified groups (R^{Ac}) expressed by -R¹-C(=O)-O-CH=CHR² bonded to a silicon atom in the molecule, and has a polyorganosiloxane structure expressed by -(R³₂SiO)ₙ-.

The vinyl-modified group (R^{Ac}) in the formula is a functional group containing a methacryloxycarbonyl group or a vinyloxycarbonyl group bonded to a silicon atom via R¹, and a spacer having a certain chain length, and the vinyl-modified organopolysiloxane of the present invention has 2 or more, preferably 2 to 20, more preferably 2 to 15 of these vinyl-modified groups. Note that the bonding site of the vinyl-modified group (R^{Ac}) may be the side chain or the terminal of the organopolysiloxane, but in the case of transvinylation of the carboxylate modified group described below, organopolysiloxanes having vinyl-modified groups (R^{Ac}) on a side chain site may be easier to synthesize due to the precursor raw materials.

In the formula, R¹ is a divalent organic group with 4 to 20 carbon atoms, a methacryloxycarbonyl group or a vinyloxycarbonyl group, and a functional group that serves as a spacer between Si atoms. If the length is too short, biodegradability may decrease, especially when used as a raw material for biodegradable polymers, and if the length is too long, the polymer or copolymer obtained by radical polymerization of the vinyl-modified organopolysiloxane may lose the feel during use and texture unique to silicone, and flexible properties may not be obtained. This R¹ is preferably an alkylene group with 4 to 20 carbon atoms, and an alkylene group with 6 to 15 carbon atoms is particularly preferred.

In the formula, R² is a hydrogen atom or a methyl group, and is a functional group that provides a vinyloxycarbonyl or methacryloxycarbonyl group.

In the formula, n is the number of repeating units of diorganosiloxane units, n is a number in a range of 1 to 1000, and n may be a number in a range of 50 to 750, from the viewpoint of flexibility of the resulting radical polymerizable polymer or copolymer, and the like. Furthermore, R³ is an alkyl group with 1 to 20 carbon atoms unsubstituted or substituted by a halogen atom, an aryl group with 6 to 22 carbon atoms, or a hydroxyl group, and industrially may be a methyl or phenyl group.

Such vinyl-modified organopolysiloxanes are preferably dimethylpolysiloxanes having a vinyl-modified group (R^{Ac}) in the side chain, and a preferable example is a vinyl-modified organopolysiloxane expressed by the following structural formula:

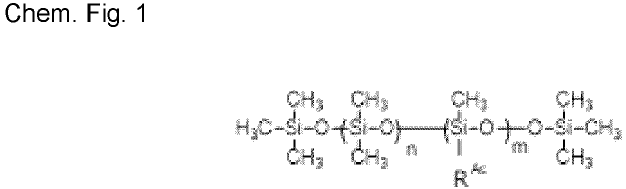

Herein, in the formula, n is a number within a range of 1 to 1000 and is preferably a number within a range of 50 to 750. m is a number in a range of 3 to 100, more preferably a number in a range of 3 to 20, or a number in a range of 3 to 15. Furthermore, R^{Ac} is a vinyl-modified group bonded to a silicon atom as described above.

### Synthesis method by transvinylation

These vinyl-modified organopolysiloxanes have relatively long-chain functional groups as spacers between Si atoms, and are obtained by reacting a precursor compound having a carboxylate modified group expressed by -R¹-C(=O)-OH (wherein R¹ is a divalent organic group with 4 to 20 carbon atoms) bonded to a silicon atom with vinyl acetate in the presence of a palladium metal catalyst.

More specifically, the method for producing the vinyl-modified organopolysiloxane according to the present invention includes:
a step of using a vinyl acetate in the presence of a palladium metal catalyst to perform a transvinylation reaction of a carboxylate-modified organopolysiloxane having 2 or more carboxylate-modified groups expressed by

   -R¹-C(=O)-OH
(where R¹ is a divalent organic group having 4 to 20 carbon atoms), bonded to a silicon atom in each molecule; and
having a polyorganosiloxane structure expressed by

   -(R³₂SiO)ₙ-
(where R³ is an alkyl group with 1 to 20 carbon atoms, either unsubstituted or substituted with a halogen atom, an aryl group with 6 to 22 carbon atoms, or a hydroxyl group, and n is a number in a range 1 to 1000).

The carboxylate-modified organopolysiloxane is a precursor raw material, and the linking group (spacer) between the Si atom and the carboxylate-modified group may be a divalent organic group, which is the aforementioned R¹. In this production method, the vinyl-modified organopolysiloxanes can be obtained in an industrially simple and inexpensive manner if the carboxylate-modified silicone raw material to be transvinylated into vinyl-modified organopolysiloxanes can be prepared.

Vinyl carboxylate compounds are the raw materials for transvinylation of carboxylate-modified groups, and provide vinyl-modified groups (R^{Ac}) in the presence of a palladium metal catalyst such as palladium acetate, palladium chloride, and palladium black. Vinyl carboxylate compounds are not restricted as long as they can be transvinylated. Examples include vinyl acetate, vinyl pivalate, vinyl butyrate, vinyl propionate, and the like, with vinyl acetate being particularly suitable because of the low cost and excellent reactivity. Furthermore, palladium metal catalysts may also be used in combination with ligands for heavy metals such as 1,10-phenanthroline, or the like.

The reaction may be carried out in an organic solvent, or in a liquid vinyl carboxylate compound such as vinyl acetate, or the like. Although the reaction conditions are not restricted, the terminal group of the carboxylic acid is preferably converted to a vinyl ester structure through a transvinylation reaction by heating in a range from room temperature to 100°C with stirring and N₂ ventilation. Reaction times generally range from several hours to several tens of hours, depending on the reaction scale and reaction temperature.

[Use as a starting raw material for radical polymerizable polymer or radical polymerizable copolymer]

The vinyl-modified organopolysiloxane of the present invention is radical polymerizable, and is expected to be biodegradable in the resulting radical polymerizable polymer or radical polymerizable copolymer. These radical polymerizable polymers or radical polymerizable copolymers can be obtained by using the vinyl-modified organopolysiloxane of the present invention alone or in combination with one or more type of radical polymerizable monomer in the presence of a radical initiator in a radical polymerization reaction.

A conventionally known compound commonly used in radical polymerization methods is used as the radical initiator, and specific examples include dimethyl 2,2'-azobis(isobutyrate), 2,2'-azobis(isobutyronitrile), 2,2'-azobis(2-methylbutyronitrile), 2,2'-azobis(2,4-dimethylvaleronitrile), and other azo-based compounds; benzoyl peroxide, lauroyl peroxide, tert-butylperoxy benzoate, tert-butylperoxy-2-ethylhexanoate, tert-hexylperoxy-2-ethylhexanoate, and other organic peroxides; and potassium persulfate, sodium persulfate, ammonium persulfate, and the like. One type of these radical initiators may be used alone, or two or more types may be mixed and used together.

The amount of radical initiator used should be in a range of 0.1 to 5 parts by mass relative to 100 parts by mass of the total of the radical polymerizable components including the vinyl-modified organopolysiloxane.

Herein, when oil-soluble azo compounds are used, the radical polymerizable polymer or radical polymerizable copolymer can be obtained without the need for a solvent. On the other hand, if the radical initiator is a water-soluble persulfate such as potassium persulfate or the like, the extreme ease of adding and reacting is advantageous if the crosslinking reactive silicone emulsified particles made by emulsifying in water the crosslinking reactive silicone composition by the radical polymerization reaction described below are subjected to a crosslinking reaction in water. Furthermore, upon completion of the radical polymerization reaction, it is particularly desirable to add aminomethylpropanediol or the like in a range of 0.1 to 5 parts by mass, for the purpose of stopping the reaction and neutralizing the solution by pH adjustment.

A chain transfer agent can be optionally added during the polymerization reaction using the aforementioned radical initiator. Specific examples of the chain transfer agent include mercapto compounds such as 2-mercaptoethanol, butyl mercaptan, n-dodecyl mercaptan, 3-mercaptopropyl trimethoxysilane, polydimethylsiloxanes having a mercaptopropyl group, and the like; and halides such as methylene chloride, chloroform, carbon tetrachloride, butyl bromide, 3-chloropropyl trimethoxysilane, and the like.

Examples of other radical polymerizable monomers include acrylic ester monomers or methacrylic ester monomers with 4 to 13 carbon atoms, and may be acrylic esters or methacrylic esters with 4 to 10 carbon atoms. Typical examples include methyl (meth)acrylate, ethyl (meth)acrylate, and n-propyl (meth)acrylate.

On the other hand, a polyfunctional vinyl-based monomer can also be used, and examples include trimethylolpropane tri(meth)acrylate, pentaerythritol tri(meth)acrylate, ethylene glycol di(meth)acrylate, tetraethylene glycol di(meth)acrylate, polyethylene glycol di(meth)acrylate, 1,4-butanediol di(meth)acrylate, 1,6-hexanediol di(meth)acrylate, neopentyl glycol di(meth)acrylate, trimethylolpropane trioxyethyl (meth)acrylate, tris(2-hydroxyethyl) isocyanurate di(meth)acrylate, tris(2-hydroxyethyl)isocyanurate tri(meth)acrylate, styryl group-capped polydimethyl siloxane, and other such unsaturated group-containing silicone compounds, and the like.

However, with the radical polymerizable copolymer of the present invention, the other radical polymerizable monomer is preferably vinyl acetate or a (meth)acryl-modified polycaprolactone compound. The inclusion of these components as raw materials may improve the biodegradability of the resulting radical polymerizable copolymer.

For example, the radical polymerizable copolymer of the present invention may include vinyl acetate as a starting raw material. The structure derived from these monomer components may improve the biodegradability of the resulting radical polymerizable copolymer, in addition to oil absorption and blending stability.

For example, the radical polymer of the present invention includes, as starting raw material, a (meth)acryl-modified polycaprolactone compound having in the molecule two or more modified polycaprolactone structures expressed by the following structural formula (1): 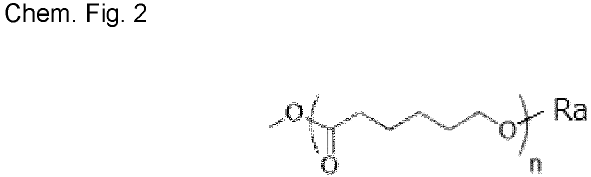 (1)The structure derived from these monomer components may improve the biodegradability of the resulting radical polymerizable copolymer, in addition to the texture and the feel during use.

n in the formula represents the number of repeating caprolactone units {-C(=O)-C₅H₁₀-O-} in the structure, and is a number in a range of 1 to 5, and may be a number in a range of 1 to 3. Furthermore, Ra is a (meth)acryl terminal group expressed by -C(=O)-R¹-CR²=CH₂. Herein, R¹ is a chemical bond between CH and C(=O) or a divalent organic group with 1 to 20 carbon atoms, and may be a simple linkage structure as in "-C(=O)-CH=" or an alkylene group with 1 to 20 carbon atoms expressed by "-C(=O)-CₘH₂ₘ-CH=" (m is a number in the range 1 to 20), such as CₘH₂ₘ (note that m is 0 when R¹ is a chemical bond between CH and C(=O)). Furthermore, R² is a hydrogen atom or a methyl group, and provides an acryl-modified group or a methacryl-modified group.

More specifically, the (meth)acryl-modified polycaprolactone compound of the present invention may be a compound expressed by one or more structural formulas selected from the following structural formulas (1-1) to (1-3). Note that these compounds have two, three, or four modified polycaprolactone structures expressed by the following structural formula (1).

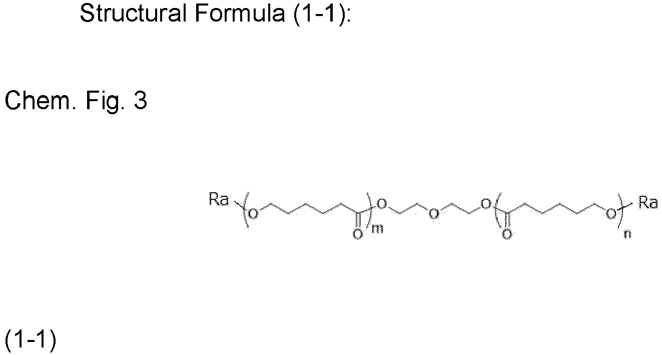

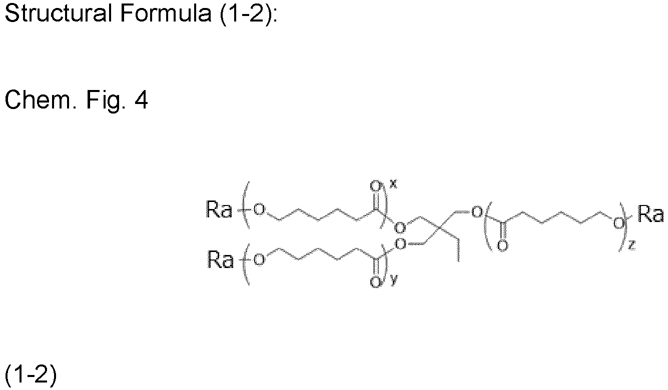

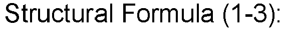

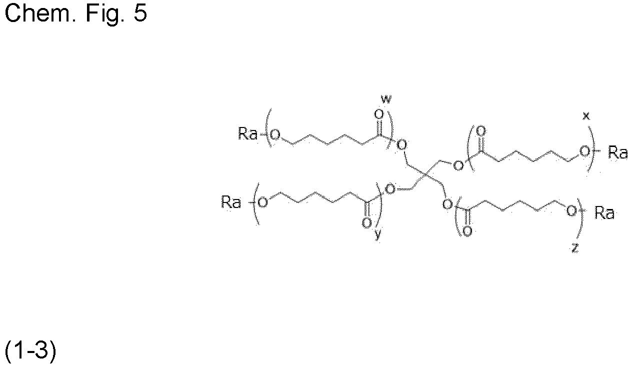

In the formula, Ra is synonymous with the groups described above. In structural formula (1-1), "m" and "n" are each a number in a range of 1 to 5, while m + n is a number in a range of 2 to 20, or m + n is a number in a range of 2.5 to 10, or can be a number in a range of 3 to 7.
In structural formulas (1-2) and (1-3), w, x, y, and z may each independently be a number in a range of 1 to 5, x + y + z may be a number in a range of 3 to 20, and w + x + y + z may be a number in a range of 4 to 20. Furthermore, x + y + z may be a number in a range of 3 to 7, and w + x + y + z may be a number in a range of 4 to 10.

These (meth)acryl-modified polycaprolactone compounds can be obtained by reacting a precursor polycaprolactone compound having a polyol terminal structure with a (meth)acryloyl chloride compound in the presence of a basic catalyst.

Radical polymerizable polymers or radical polymerizable copolymers containing a vinyl-modified organopolysiloxane as at least a portion of the starting material of the present invention may be in gel or particle form. In particular, they may be silicone elastomer particles as described below.

### Silicone elastomer particles

The vinyl-modified organopolysiloxane of the present invention is useful as a starting raw material for silicone elastomer particles and is obtained by radical polymerization of (A) the aforementioned vinyl-modified organopolysiloxane and (B) one or more types of radical polymerizable monomer in the presence of (C) a radical initiator.

The silicone elastomer particles according to the present invention are preferably obtained by curing crosslinking reactive silicone emulsified particles by a crosslinking reaction. Particularly suitably, the silicone elastomer particles of the present invention are defined by the manufacturing process, and include at least (A) the aforementioned vinyl-modified organopolysiloxane, (B) one or more types of radical polymerizable monomer, and (C) a radical polymerization initiator, and the silicone elastomer particles are made by crosslink reacting in water the crosslinkable silicone emulsified particles made by emulsifying in water a crosslinkable silicone composition that is crosslinkable using a radical polymerization reaction.

From the viewpoint of improving the biodegradability, feel during use, oil absorption, and the like, the silicone elastomer particles of the present invention preferably use the aforementioned vinyl-modified organopolysiloxane and one or more radical polymerization monomer selected from vinyl acetate and (meth)acryl-modified polycaprolactone compound as the (B) component, either in whole or in part.

Furthermore, silicone elastomer particles obtained via such a manufacturing process may further improve the appearance, spreadability, and tactile sensation of the cosmetic material, particularly when used as a cosmetic raw material, and particles obtained via this manufacturing method tend to be more suitable for solving the problem of the present invention. Thus, one suitable form for achieving the technical effect of the present invention can be and is suitably defined by the manufacturing process.

The crosslinking reactive silicone composition above may contain one or more polymerization inhibitors from the perspective of preventing unintended side reactions and the like. For example, one or more types selected from hindered phenol-based polymerization inhibitors, hydroquinone-based polymerization inhibitors, and catechol-based polymerization inhibitors may be included. The amount used can be selected as appropriate, but the total concentration of the polymerization inhibitor is preferably 50 ppm by mass or less, and more preferably 30 ppm by mass or less, with respect to the sum of components (A) to (C) above.

The crosslinking reactive silicone composition may include a component other than the components above to the extent that the technical effects of the present invention are not impaired. For example, the composition may include: n-hexane, cyclohexane, n-heptane, or other aliphatic hydrocarbons; toluene, xylene, mesitylene, or other aromatic hydrocarbons; tetrahydrofuran, dipropyl ether, or other ethers; an organic solvent such as acetone, methyl ethyl ketone, methyl isobutyl ketone, other ketones, or the like; a phenolic antioxidant, quinone antioxidant, amine antioxidant, phosphorus antioxidant, phosphite antioxidant, sulfur-based antioxidant, thioether antioxidant, or other antioxidant; a triazole light stabilizer, benzophenone light stabilizer, or other light stabilizer; a phosphate ester flame retardant, halogen flame retardant, phosphorus flame retardant, antimony flame retardant, or other flame retardant; one or more antistatic agents including cationic surfactants, anionic surfactants, non-ionic surfactants, and the like; a dye; a pigment; or the like.

The silicone elastomer particles of the present invention may optionally further have: (A) a structure in which part or all of a surface thereof is coated by one or more substance selected from organopolysiloxane resins, silica, and other silicone elastomer particles; (B) a mesoporous structure; (C) a structure containing an oil agent that is liquid at 40°C; and (D) a structure crosslinked by a silalkylene group having 2 to 20 carbon atoms, and a combination of arbitrary components providing these structures may be used.

### Applications for radical polymerizable polymers or radical polymerizable copolymers

The radical polymerizable polymer or radical polymerizable copolymer (including the silicone elastomer particles described above) of the present invention is useful as a raw material for cosmetic materials, and when blended in a cosmetic composition or the like, the cosmetic material will be flexible with even more excellent feel during use and texture, and will have remarkable stability in handling, storage and blending as a raw material for cosmetic materials, and the like.

Similarly, the radical polymerizable polymer or radical polymerizable copolymer (including the silicone elastomer particles described above) of the present invention is also extremely useful as an organic resin additive. Specifically, the radical polymerizable polymer or radical polymerizable copolymer of the present invention have superior uniform dispersibility in organic resins and, if desired, excellent stress relief properties, and the like. In addition, they may have very excellent handling workability and storage stability because aggregation is less likely to occur even after long-term storage. Furthermore, a member, paint film, or coating film obtained by curing an organic resin containing the silicone elastomer particles has improved flexibility (including the softness of a coating layer), durability, and adhesion and conformability to the substrate, is particularly pliable, and has superior thermal shock resistance. Therefore, it is extremely useful as a highly functional organic resin, paint, or coating agent for use in electronic materials.

### As Eco-Friendly Material

As described above, unlike conventional non-biodegradable polymeric materials, the radical polymerizable polymers or radical polymerizable copolymers of the present invention are biodegradable in a biodegradable environment, where the cross-linked structures formed between silicon atoms are at least partially cleaved, and biodegradability properties are anticipated in conjunction with generation of non-crosslinked polyorganosiloxane. Therefore, use is possible as an "eco-friendly" cosmetic raw material with low environmental burden and environmental risk that complies with regulations on microplastics and the like, and appeal as a biodegradable and "eco-friendly" material is expected for users and consumers who are concerned about global environmental impact.

### Examples

### Example 1: Vinyl-modified organopolysiloxane No. 1

75.2 parts by weight of a carboxylate-modified silicone oil (molecular weight: 31649.36) expressed by structural formula: 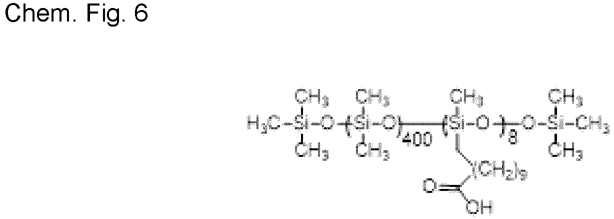 and 24.6 parts by weight of vinyl acetate (made by Fujifilm Wako, molecular weight: 86.09) were added to a four-neck round bottom flask equipped with a mechanical stirrer, thermometer and oil bath. After bubbling under nitrogen (N₂) flow for 5 min while stirring at 200 rpm, 0.1 wt.% palladium acetate (catalyst, Fujifilm Wako, molecular weight: 224.5) and 0.1 wt.% 1,10-phenanthroline (ligand, Fujifilm Wako, molecular weight: 180.21) were added under N₂ aeration.

After stirring at room temperature, the temperature was raised to 60°C and allowed to react for 24 hours (Overnight).

After the reaction was completed, ¹³C-NMR analysis confirmed the disappearance of the carboxylate derived from the raw material, and the remaining vinyl acetate was distilled off under reduced pressure with N₂ bubbling. Finally, the catalyst was removed by filtration using activated carbon-added filter paper.

After filtration, a clear silicone oil was obtained. The structure was confirmed by H-NMR and ¹³C-NMR, and it was confirmed that vinyl-modified organopolysiloxane No. 1 with the following vinyl ester structure was formed.

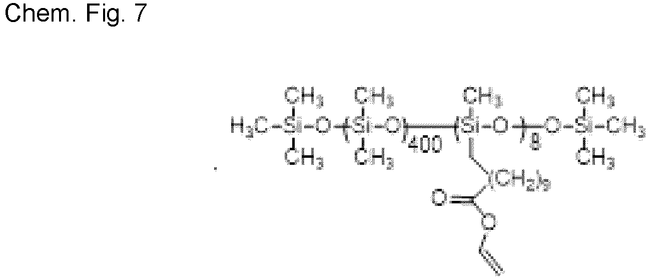

### Example 2: Synthesis of gel composition of radical polymerizable polymer

96 parts by weight of vinyl-modified organopolysiloxane No. 1 were placed in a three-neck round bottom flask equipped with a stirrer, thermometer and oil bath, and then bubbling was performed using N₂ while stirring at 200 rpm. After bubbling for 5 minutes, 4 parts by weight of oil-soluble azo polymerization initiator "Trade name V-601" dimethyl (2,2'-azobis(isobutyric acid), manufactured by Fujifilm Wako, molecular weight: 230.26) was added. While stirring the raw material, the temperature was increased to 70°C. After 3 hours, a gel-like material, a single radical polymer of vinyl-modified organopolysiloxane No. 1, formed in the flask.

### Examples 3, Comparative Example 1: Production of silicone elastomer particles

Example 3 presents production examples of silicone elastomer particles obtained by using the vinyl-modified organopolysiloxane No. 1 and a (meth)acryl-modified caprolactone compound as raw materials. Note that Comparative Example 1 is non-silicone-based polymer particle obtained using only a (meth)acrylic-modified polycaprolactone compound as a raw material.

### Example 3

Vinyl-modified organopolysiloxane No. 1 and a (meth)acryl-modified polycaprolactone compound expressed by the following structural formula: 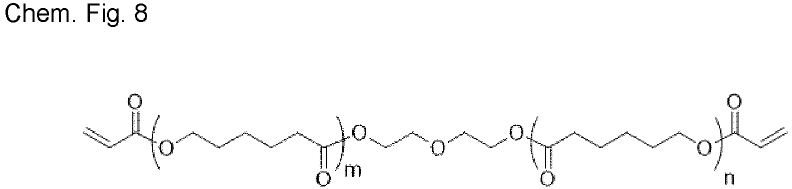 (where m + n = 3.7)
were mixed uniformly at a mass ratio of 30:70 at room temperature, and vegetable oil (produced by AAK) was added and mixed in an amount to make 20% of the total composition. Next, this composition was dispersed in an aqueous solution at 25°C containing 0.24 parts by mass of GOHSENOL EG-05C and 0.47 parts by mass of GOHSENOL EG-18P in 46 parts by mass of pure water, and after uniform emulsification using a colloid mill, 300 parts by mass of pure water was added. After heating in a 1 L flask to 60°C, an aqueous solution of 0.5 g potassium persulfate (manufactured by Sigma-Aldrich) dissolved in 9.5 g water was added dropwise over one minute. The emulsion was stirred at 60°C for three hours at 100 rpm to perform radical polymerization to prepare a uniform aqueous suspension of silicone rubber particles. The aqueous suspension was then filtered and washed with 200 mL of ethanol and 100 mL of acetone. The residue was dried in an oven at 70°C for 3 hours to obtain silicone elastomer particles. The average secondary particle diameter of the resulting silicone elastomer particles was 119 µm.

### Comparative Example 1

Silicone elastomer particles were obtained in the same manner as in Example 3, except that a polyorganosiloxane was not used and 100 parts by mass of the (meth)acryl-modified polycaprolactone compound was used as the raw material in Example 3. The average secondary particle diameter of the resulting silicone elastomer particles was 70.0 µm.

The average primary and secondary particle diameters of each particle obtained from the above Example 3 and Comparative Example 1 are summarized in Table 1 below.

**Table 1**

| | Average primary particle diameter (µ m) | Average secondary particle diameter (µ m) |
|---|---|---|
| Example 3 | 2.32 | 119 |
| Comparative example 1 | 2.91 | 70.0 |

### Cosmetic Material Formulation Example

The following are formulation examples of cosmetic materials of the present invention that can contain silicone elastomer particles, which is one aspect of the present invention. However, the present invention is not limited thereto.

The texture of the silicone elastomer particles of Example 3 and the powder of Comparison Example 1 were compared and evaluated by applying to the inside of the arm of panelists.

The silicone elastomer particles of Example 3 were softer and more slippery than the powder in Comparison Example 1.

### Example 4 and Comparative Example 5

Panelists compared and evaluated the feel during use of loose powder in which the silicone elastomer particles in the compositions listed in Table 3 were used.

### Evaluation of Tactile Sensation

The slipperiness of the samples applied to inner forearms of 18 panelists was evaluated using the criteria in Table 2 below.

**Table 2**

| Evaluation results | Evaluation Indicators |
|---|---|
| Good | 12 or more of 18 respondents said the slipperiness was favorable |
| Fair | 7 to 11 of 18 respondents said the slipperiness was favorable |
| Poor | 6 or fewer of 18 respondents said the slipperiness was favorable |

**Table 3**

| Phase | Component | Product name and supplier | Example 4 | Comparative example 2 |
|---|---|---|---|---|
| A | Silicone elastomer particles of Example 3 | | 10 | |
| | Elastomer particles of Comparative Example 1 | | | 10 |
| | Bis(hydroxyethylpropyl) dimethicone | DOWSIL^{™} 5562 Carbinol Fluid | 6 | 6 |
| B | Talc | JA-46R available from ASADA MILLING CO., LTD. | 76 | 76 |
| | Silylated silica | DOWSIL^{™} VM-2270 Aerogel Fine Particle | 2 | 2 |
| | Kaolin | | 1.5 | 1.5 |
| | Titanium oxide | SI Titanium CR-50 available from Miyoshi Kasei, Inc. | 3.92 | 3.92 |
| | Iron oxide yellow | SI-YELLOW-LLXLO available from Miyoshi Kasei, Inc. | 0.46 | 0.46 |
| | Iron oxide red | SA-Bengara Cloisonne available from Miyoshi Kasei, Inc. | 0.09 | 0.09 |
| | Iron oxide black | SA-Black BL-100 available from Miyoshi Kasei, Inc. | 0.02 | 0.02 |
| Evaluation of tactile sensation | | | Good | Poor |

### (Method of preparation)

1. Phase A is mixed.
2. Phase B is mixed.
3. Phases A and B are stirred until uniform.

As shown in Table 3, loose powder using the silicone elastomer particles of the present invention (Example 3) was evaluated to have relatively favorable slipperiness, as compared to loose powders that use other particles (Comparative Example 1).

## Claims

1. A vinyl-modified organopolysiloxane having 2 or more vinyl-modified groups (R^{Ac}) expressed by
-R¹-C(=O)-O-CH=CHR²
(wherein R¹ is a divalent organic group having 4 to 20 carbon atoms and R² is a hydrogen atom or a methyl group), bonded to a silicon atom in each molecule; and
having a polyorganosiloxane structure expressed by
-(R³₂SiO)ₙ-
(wherein R³ is an alkyl group with 1 to 20 carbon atoms, either unsubstituted or substituted with a halogen atom, an aryl group with 6 to 22 carbon atoms, or a hydroxyl group, and n is a number in the range 1 to 1000).

2. The vinyl-modified organopolysiloxane according to claim 1, wherein in the vinyl-modified group (R^{Ac}) bonded to a silicon atom, R¹ is an alkylene group having 4 to 20 carbon atoms, and which contains 3 or more vinyl-modified groups (R^{Ac}) bonded to a silicon atom in each molecule.

3. The vinyl-modified organopolysiloxane according to claim 1 or claim 2, expressed by the following structural formula: 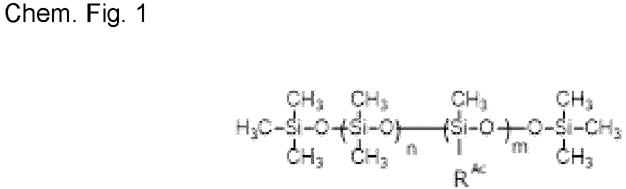 (in the formula, n is a number in a range 1 to 1000, m is a number in a range 3 to 100, and R^{Ac} is a vinyl-modified group bonded to a silicon atom as described above).

4. A method for producing the vinyl-modified organopolysiloxane according to any one of claims 1 to 3, comprising:
a step of using a vinyl carboxylate compound in the presence of a palladium metal catalyst to perform a transvinylation reaction of a carboxylate-modified organopolysiloxane having 2 or more carboxylate-modified groups expressed by
-R¹-C(=O)-OH
(where R¹ is a divalent organic group having 4 to 20 carbon atoms), bonded to a silicon atom in each molecule; and
having a polyorganosiloxane structure expressed by
-(R³₂SiO)ₙ-
(where R³ is an alkyl group with 1 to 20 carbon atoms, either unsubstituted or substituted with a halogen atom, an aryl group with 6 to 22 carbon atoms, or a hydroxyl group, and n is a number in a range 1 to 1000).

5. A radical polymerizable polymer or radical polymerizable copolymer, comprising: the vinyl-modified organopolysiloxane according to any one of claims 1 to 3 as at least a portion of starting raw material.

6. A gel-like or particulate material containing the radical polymerizable polymer or radical polymerizable copolymer of claim 5.

7. Silicone elastomer particles, comprising: the vinyl-modified organopolysiloxane according to any one of claims 1 to 3 as at least a portion of starting raw material.

8. Silicone elastomer particles according to claim 7, comprising at least:
(A) the vinyl-modified organopolysiloxane according to any one of claims 1 to 3;
(B) one or more radical polymerizable monomer; and
C) a radical initiator;
made by performing a crosslinking reaction in water of crosslinking reactive silicone emulsified particles obtained by emulsifying in water a crosslinking reactive silicone composition using a radical polymerization reaction.

9. Silicone elastomer particles according to claim 7, wherein component (B) contains one or more radical polymerizable monomer selected from vinyl acetate and (meth)acrylic- modified polycaprolactone compounds, and is biodegradable.

10. A cosmetic raw material comprising: the radical polymerizable polymer or radical polymerizable copolymer of claim 5.

11. A cosmetic material composition, comprising: the radical polymerizable polymer or radical polymerizable copolymer of claim 5.

12. An organic resin additive, comprising: the radical polymerizable polymer or radical polymerizable copolymer of claim 5.

13. An organic resin, comprising: the radical polymerizable polymer or radical polymerizable copolymer of claim 5.
